# EUROPEAN PATENT SPECIFICATION

(11) **EP 1 409 014 B1**
(45) Date of publication and mention of the grant of the patent: **21.03.2007**
(21) Application number: 02784987.6
(22) Date of filing: 18.12.2002
(51) Int. Cl.: A61K 39/39, A61K 48/00, A61K 39/155

(54) **IMMUNOGENIC COMPOSITIONS COMPRISING AN ANTIGEN AND A PURIFIED M PROTEIN FROM RESPIRATORY SYNCYTIAL VIRUS**
IMMUNOGENE ZUSAMMENSETZUNGEN DIE EIN ANTIGEN UND EIN M PROTEIN DES RESPIRATORISCHEN SYNZYTIALVIRUSES ENTHALTEN
COMPOSITIONS IMMUNOGENES COMPRENANT UN ANTIGENE ET UNE PROTEINE M PURIFIEE PROVENANT DU VIRUS RESPIRATOIRE SYNCYTIAL

(30) Priority: 20.12.2001 US 341422 P
(43) Date of publication of application: 21.04.2004
(73) Proprietor: Aventis Pasteur Limited, Toronto, Ontario M2R 3T4 (CA)
(72) Inventor: BARBER, Brian, Hawthorne, NY 10532-1533 (US); CATES, George, Richmond Hill, Ontario L4C 3T5 (CA); PARRINGTON, Mark, Bradford, Ontario L3Z 1Z4 (CA); SAMBHARA, Suryprakash, 1600 Clifton Road, Atlanta, GA 30333 (US)
(74) Representative: Lawrence, Malcolm Graham
(86) International application number: PCT/CA2002/001953
(87) International publication number: WO 2003/053464

(56) References cited:
- WO-A-98/02180
- WO-A-98/02457
- WO-A-99/30733
- NEUZIL K M ET AL: "Adjuvants influence the quantitative and qualitative immune response in BALB/c mice immunized with respiratory syncytial virus FG subunit vaccine" VACCINE, BUTTERWORTH SCIENTIFIC. GUILDFORD, GB, vol. 15, no. 5, 5 April 1997 (1997-04-05), pages 525-532, XP004059891 ISSN: 0264-410X

## Description

### FIELD OF THE INVENTION

The present invention relates to the field of immunology and is particularly concerned with enhancing an immune response to an antigen.

### BACKGROUND OF THE INVENTION

Adjuvants have been used for many years to improve the host immune response to antigens of interest in vaccines, especially subunit or component vaccines comprised of recombinant proteins. Adjuvants are immunomodulators that are typically non-covalently linked to antigens and are formulated to enhance the host immune response. Examples include aluminum hydroxide and aluminum phosphate (collectively commonly referred to as alum). While little or no systemic toxicity is observed with alum, its use is associated with local reactions such as erythema, subcutaneous nodules, contact hypersensitivity and granulomatous inflammation. Such local reactions may be of particular concern in the context of frequent, e.g., annual immunizations.

Adjuvants enhance the immunogenicity of an immunogen but are not necessarily immunogenic themselves. Adjuvants have been identified that enhance the immune response to antigens delivered parenterally. Some of these adjuvants are toxic, however, and can cause undesirable side-effects making them unsuitable for use in humans and many animals. Indeed, only aluminum hydroxide and aluminum phosphate (collectively commonly referred to as alum) are routinely used as adjuvants in human and veterinary vaccines. To efficiently induce humoral immune responses (HIR) and cell-mediated immunity (CMI), immunogens (antigens) are often emulsified in adjuvants. Many adjuvants are toxic, inducing granulomas, acute and chronic inflammations (Freund's complete adjuvant, FCA), cytolysis (saponins and pluronic polymers) and pyrogenicity, arthritis and anterior uveitis (LPS and MDP). Although FCA is a potent adjuvant and widely used in research, it is not licensed for use in human or veterinary vaccines because of its toxicity.

Human Respiratory Syncytial Virus (RSV) is a major cause of respiratory tract infections. Globally, 65 million infections occur every year resulting in 160,000 deaths (ref. 1; a list of references appears at the end of the disclosure.) In the USA alone 100,000 children, may require hospitalization for pneumonia and bronchiolitis caused by RSV in a single year (refs. 2). Providing inpatient and ambulatory care for children with RSV infections costs in excess of $340 million annually in the USA.

RSV is a major cause of serious lower respiratory illness in elderly and immunocompromised adults (refs. 3). Outbreaks in nursing or retirement homes are well documented (ref. 3) and a significant proportion of disease involving the lower respiratory tract in outbreaks were associated with mortality. Approximately 7 % of hospitalized community acquired pneumonias have been attributed to RSV (ref. 4). Mortality due to RSV may exceed that due to influenza by 60 to 80% (ref. 5) The annual costs attributed to hospitalizations for RSV pneumonia in the elderly in the USA has been conservatively estimated at between $150 to $680 million (ref. 6). An RSV vaccine could therefore play an important role in lessening morbidity and mortality in the elderly and decreasing health care costs.

RSV is an enveloped RNA virus of the family paramyxoviridae and of the genus pneumovirus. The structure and composition of RSV has been elucidated and is described in detail in the textbook "Fields Virology", Fields, B.N. Raven Press, N.Y. (1996), pp 1313-1351 "Respiratory Syncytial Virus" by Collins, P., McIntosh, K., and Chanock, R.M. (ref. 7).

Cross neutralization studies have shown that RSV isolates can be classified into two major antigenic groups, designated A and B. (ref. 8) The G glycoprotein shows the greatest divergence between groups showing 53% amino acid homology between RSV A and B. (ref. 9)

The two major protective antigens of RSV are the envelope fusion (F) and the attachment (G) glycoproteins (ref. 10). The F protein is synthesized as an about 68 kDa precursor molecule (Fo) which is proteolytically cleaved into disulfide-linked F1 (about 48 kDa) and F2 (about 20 kDa) polypeptide fragments (ref. 11). The G protein (about 33 kDa) is heavily O-glycosylated giving rise to a glycoprotein of apparent molecular weight of about 90 kDa (ref. 12). Two broad subtypes of RSV have been defined A and B (ref. 13). The major antigenic differences between these subtypes are found in the G glycoprotein while the F glycoprotein is more conserved (refs. 14).

Antibodies directed against the F protein or against the G protein can neutralize the virus. Antibodies to the F protein block the spread of the virus between cells.

In addition to the antibody response generated by the F and G glycoproteins, human cytotoxic T cells produced by RSV infection have been shown to recognize the RSV F protein, matrix protein (M), 22k protein (M22) nucleoprotein (N), small hydrophobic protein (SH), and the non-structural protein (1b.) (ref 15). The human RSV M gene coding for the matrix protein has been sequenced (ref 18).

WO 98/02180 describes a two-step immunization procedure against the pyramyxoviridae family of viruses using attenvated viral strains and sub unit protein preparation.

It would be desirable to enhance the immune response to an antigen, in particular an RSV antigen to provide a more efficacious vaccine.

### SUMMARY OF THE INVENTION

The present invention provides methods, compositions and uses as defined in the claims and as described below.

Accordingly, in one aspect of the present invention there is provided an immunogenic composition comprising purified M protein from respiratory syncytial virus or at least one immunoeffective fragment thereof and a nucleic acid vector encoding a respiratory syncytial virus antigen or at least one immunogenic fragment thereof, wherein said M protein or immunoeffective fragment is provided in an amount to provide an enhanced immune response to said respiratory syncytial virus antigen in a host having a pre-existing respiratory syncytial virus M-specific immune response, wherein the nucleic acid vector comprises an antigen-encoding portion and a promoter to effect expression of the antigen-encoding portion in the host.

In accordance with another aspect of the present invention, there is provided a method of making an immunogenic composition comprising providing purified M protein from respiratory syncytial virus or at least one immunoeffective fragment thereof and a nucleic acid encoding a respiratory syncytial virus antigen, wherein said M protein or immunoeffective fragment is provided in an amount to provide an enhanced immune response to said respiratory syncytial virus antigen in a host having a pre-existing respiratory syncytial virus M-specific immune response.

In a further aspect of the present invention there is provided the use of a purified M protein or immunogenic component thereof from respiratory syncytial virus as an adjuvant for the preparation of an immunogenic composition for use in a host having a pre-existing respiratory syncytial virus M-specific immune response, wherein said composition comprises a respiratory syncytial virus antigen and said M-protein.

In a further aspect of the present invention there is provided the use of a purified M protein from respiratory syncytial virus, or immunoeffective fragments thereof, for the manufacture of a medicament comprising a nucleic acid vector encoding a respiratory syncytial virus antigen for enhancing the immune response to a respiratory syncytial virus antigen in a host having a pre-existing respiratory syncytial virus M-specific immune response.

The hosts protected against disease caused by RSV include humans and the immunogenic compositions of the invention can be used in methods of immunization and protection of hosts against disease caused by infection by RSV. The immunogenic compositions of the invention can be administered to susceptible hosts. The hosts may be elderly humans or other humans previously exposed to RSV M protein and immunologically primed to respond to the immunization.

### BRIEF DESCRIPTION OF DRAWINGS

The present invention will be further understood from the following description with reference to the drawings, in which:
Figure 1 is a graphical representation of the IFN-γ secretion from spleen cells of mice primed with live RSV and boosted with various RSV antigens encoded by a viral vector (NYVAC).
Figure 2 is a graphical representation of the CTL response from the viral vectors of figure 1, showing the percentage specific lysis of target cells.
Figure 3 shows the T-cell response in mice to other RSV antigens when RSV M protein is co-administered.
Figure 4 shows the enhanced antibody response in mice to other RSV antigens when RSV M protein is co-administered.

### GENERAL DESCRIPTION OF THE INVENTION

As discussed above, the present invention provides an enhanced response to respiratory syncytial virus (RSV) antigens by the use of respiratory syncytial virus M protein. While the inventors have not identified the exact mechanism of the enhanced immune response to an antigen due to the RSV M protein, it is thought that the M protein mobilizes T-cell help. In a preferred embodiment RSV antigens to be encoded by the nucleic acid vector in the vaccines may include the RSV F, G and M22 proteins. The proteins can be isolated from RSV by, for example, immunoaffinity purification, ion-exchange or other biochemical procedures as described in, for example, International Patent Application No. WO 94/27636 of Hancock, published December 8, 1994 or by the procedure described in US patent No. 5,194,595. The RSV proteins and immunogenic fragments thereof can be isolated from recombinant organisms that express the proteins or immunogenic fragments. The gene encoding the F protein is described in ref. 16. The gene encoding the G protein is described in ref. 17 and the gene encoding the M protein is described in ref. 18. The production of recombinant organisms expressing the RSV proteins or immunogenic fragments thereof and the identification and purification of the expressed gene products is described in, for example, US Patent No. 5,223,254. Such recombinants include any bacterial transformants, yeast transformants, cultured insect cells infected with recombinant baculoviruses or cultured mammalian cells as known in the art, for example, Chinese hamster ovary cells that express the RSV virus proteins or immunogenic fragments thereof.

The RSV proteins and immunogenic fragments thereof can also be chemically synthesized.

The fusion (F) protein may comprise multimeric fusion (F) proteins which may include, when analyzed under nonreducing conditions, heterodimers of molecular weight approximately 70 kDa and dimeric and trimeric forms thereof:
The attachment (G) protein may comprise, when analyzed under non-reducing conditions, oligomeric G protein, G protein of molecular weight approximately 95 kDa and G protein of molecular weight approximately 55 kDa.
The matrix (M) protein may comprise, when analyzed under non-reducing conditions, protein of molecular weight approximately 28 to 34 kDa.
The immunogenic compositions provided herein may be formulated as a vaccine for *in vivo* administration to a host, which may be a primate, most preferably a human host, to confer protection against disease caused by RSV. The immunogenic compositions and vaccines provided herein may comprise at least one further immunogenic material which may be an antigen from a pathogen other than RSV, such as a bacterial or viral antigen to provide a combination vaccine for protection against a plurality of diseases.

In this specification RSV M antigen is defined as purified and isolated RSV M protein or immunoeffective fragments thereof, or vectors delivering RSV M protein. Immunoeffective fragments of RSV M protein includes fragments or peptides derived from RSV M protein that are capable of eliciting the immunologic enhancement to another antigen. Purified in this specification means isolated away from other RSV proteins such that the RSV M protein represents the dominant material as assayed by SDS-PAGE or M-specific ELISA assays for example. Preferably the M protein should be greater than about 50% pure, more preferably greater than about 70% pure and most preferably greater than about 90% pure.

Pre-selected amounts of RSV M protein refers to defined amounts of the M protein, for example as measured by M-specific ELISA, that are added to another antigen such that there is an enhanced immune response to the other antigen in the host when compared to the same composition without the RSV M protein. Enhanced immune response can be measured by interferon gamma (IFN γ) secretion, a CTL response as determined by specific cell lysis, IL-5 secretion, or antibody responses as measured by specific ELISA. Immune response can also be measured by any other assay available to one skilled in the art and is not limited to those exemplified here. A further measure can be by enhanced efficacy against disease in a relevant model system. Pre-selected amounts may differ depending upon the host to which it is administered. Examples of RSV M amounts can be from about 0.1 µg to about 1,000 µg, or about 0.1µg to about 100µg. In other situations it may be preferable to administer between about 1µg to about 50µg of RSV M protein.

Pre-existing RSV M-specific response is defined as any detectable immune response to RSV M protein in the host. This includes T-cell responses as measured by cytokine production, for example IFNγ from CD8+ T-cells, as detected by cytokine specifio-ELISA assay or ELISPOT assay, both well known assays in the art. RSV M protein pre-priming induces cytokine responses to heterologous proteins including viral derived from RSV or any other virus, bacterial or tumor antigens, CTL responses to heterologous antigens delivered by a vector (bacterial, viral or nucleic acid, or antigens formulated to deliver antigens for class I presentation such as liposomes) and antibody responses in naive or previously RSVimmune population.

The RSV antigen to be encoded by the nucleic acid vector used with the RSV M protein can be isolated from the disease causing organism (pathogen), or can be recombinantly produced in any of the well known bacterial or eukaryotic expression systems. The RSV antigen is delivered by a nucleic acid vector such as non-replicating plasmid expression vectors encoding the antigen of choice, and containing a promoter to effect expression of said antigen in the host. Alternatively the nucleic acid vector can be a viral vector. Examples of non-replicating in the host viral vectors used to effect expression of said antigen are the pox vectors NYVAC and ALVAC (Ref 20) Viral vectors can also be replicating viral vectors such as recombinant alphavirus vectors as described in United States patent 6,224,879 and US patent 6,015,686.

### VACCINE PREPARATION AND USE

Immunogenic compositions, suitable to be used as vaccines, may be prepared from mixtures comprising M protein and a nucleic acid vector encoding the RSV antigen. The immunogenic composition elicits an immune response which produces antibodies, and/or cell mediated responses such as cytotoxic T-cell response to the specific RSV antigen.

Immunogenic compositions including vaccines may be prepared as injectables, as liquid solutions, suspensions or emulsions. The active immunogenic ingredients may be mixed with pharmaceutically acceptable excipients which are compatible therewith.

Such excipients may include water, saline, dextrose, glycerol, ethanol and combinations thereof. The immunogenic compositions and vaccines may further contain auxiliary substances, such as, wetting or emulsifying agents, pH buffering agents, or adjuvants to enhance the effectiveness thereof. Immunogenic compositions and vaccines may be administered parentally, by injection subcutaneous, intradermal or intramuscularly injection. Alternatively, the immunogenic compositions formulated according to the present invention, may be formulated and delivered in a manner to evoke an immune response at mucosal surfaces. Thus, the immunogenic composition may be administered to mucosal surfaces by, for example, the nasal or oral (intragastric) routes. Alternatively, other modes of administration including suppositories and oral formulations may be desirable. For suppositories, binders and carriers may include, for example, polyalkalene glycols or triglycerides. Such suppositories may be formed from mixtures containing the active immunogenic ingredient(s) in the range of about 10%, preferably about 1 to 2%. Oral formulations may include normally employed carriers, such as, pharmaceutical grades of saccharine, cellulose and magnesium carbonate. These compositions can take the form of solutions, suspensions, tablets, pills, capsules, sustained release formulations or powders and contain about 1 to 95% of the active ingredients, preferably about 20 to 75%.

The immunogenic preparations and vaccines are administered in a manner compatible with the dosage formulation, and in such amount as will be therapeutically effective, immunogenic and protective. The quantity to be administered depends on the subject to be treated, including, for example, the capacity of the individual's immune system to synthesize antibodies, and, if needed; to produce a cell-mediated immune response. Precise amount of active ingredients required to be administered depend on the judgment of the practitioner. However, suitable dosage ranges are readily determinable by one skilled in the art and may be of the order of micrograms to milligrams of the active ingredients) per vaccination. Suitable regimes for initial administration and booster doses are also variable, but may include an initial administration followed by subsequent booster administrations. The dosage may also depend on the route of administration and will vary according to the size of the host.

The concentration of the active ingredients in an immunogenic composition according to the invention is in general about 1 to 95%. A vaccine which contains antigenic material of only one pathogen is a monovalent vaccine.

### EXAMPLES

The above disclosure generally describes the present invention. A more complete understanding can be obtained by reference to the following specific Examples.

Methods of determining tissue culture infectious dose₅₀ (TCID₅₀/mL), plaque and neutralization titres, not explicitly described in this disclosure are amply reported in the scientific literature and well within the scope of those skilled in the art. Protein concentrations were determined by the bicinchoninie acid (BCA) method as described in the Pierce Manual (23220, 23225; Pierce Chemical company, U.S.A.), incorporated herein by reference.

CMRL 1969 culture medium was used for cell culture and virus growth. The cells used in this study are vaccine quality African green monkey kidney cells (VERO lot M6) obtained from Institut Merieux. The RS viruses used were the RS virus subtype A (Long and A2 strains) obtained from the American Type culture Collection (ATCC) for use in the virus neutralization assay and a recent subtype Δ clinical isolate for viral protein purification.

### Example 1:

### This example illustrates a method of purifying RSV M protein.

An RSV concentrate was pelleted by centrifugation for 30 minutes at 5,000 x g and the viral pellet was extracted with 2%Triton® X-100 in 1mM sodium phosphate pH 6.8, 300 mM NaCl by stirring for 1 hour at room temperature. The growth and harvest conditions for RSV can be found in US patent 6,020,182. The extract was centrifuged for 30 minutes at 15,000 x g and the supernatant was collected. The soluble supernatant was diluted two-fold with 1 mM sodium phosphate pH 6.8, 2% Triton® X-100 and then applied to a ceramic hydroxyapatite type II (Bio-Rad Laboratories) column equilibrated with 1 mM sodium phosphate, pH 6.8, 50 mM NaCl and 0.02% Triton® X-100. The column was washed with the same buffer and then an RSV M-rich fraction was collected by elution with the 1mM sodium phosphate pH 6.8, 300 mM NaCl and 0.02% Triton® X-100. The elution pool was concentrated using an Amicon stirred-cell concentrator and a YM-10 ultrafiltration membrane and then dialyzed overnight against 20 mM Tris, pH 8.0, 600 mM NaCl, 0.02% Triton® X-100. The dialyzed concentrate was applied to a Sephacryl® S-200 size exclusion column equilibrated with 20 mM Tris, pH 8.0, 600 mM NaCl, 0.02% Triton® X-100 and the M-rich peak was collected. Trace amounts of RSV F and RSV G glycoproteins were removed by passing the M-rich pool consecutively over an anti-F affinity column and a jacalin-Sepharose® column. The purified RSV M was concentrated using an Amicon stirred-cell concentrator and a YM-10 ultrafiltration membrane and then frozen at -70° C.

### Example 2:

### This example illustrates the T-cell response in mice to other RSV antigens when delivered by viral vector immunization.

Eight-week old BALB/c mice used in this study were primed with either PBS or 10³ pfu of live RSV intranasally. They were boosted (i.m.) 4 weeks later with 10⁷ pfu of NYVAC-M22 or NYVAC-F or NYVAC with or without RSV-M protein at 1µg/mouse in PBS as outlined in Table 1 below. Sera samples were collected to measure anti-RSV F titers. Spleens were collected 4 weeks after boosting to look for CTL activity and cytokine production. Spleen cells were restimulated in vitro with BC or BCH4 cells (persistantly infected with RSV). Supernatants were collected at 72 h and tested for IFN-γ and IL-5.

For IFN-γ detection in the culture supernatants, wells of Nunc-Immulon-Maxisorp plates were coated with 50mL of rat anti-mouse rIFN-γ antibody (Biosource) at 2 mg/mL in 0.05M Carbonate/Bicarbonate buffer (pH 9.6), overnight at room temperature. Wells were blocked with 200mL of 10% FBS in PBS for 1 hour at room temperature, followed by 2 washes in 0.05% Tween 20 in PBS (washing buffer). Samples and reference standards were diluted in sample buffer (0.05% Tween 20 in RPMI 1640) and 100mL of each sample was added to the wells. Plates were incubated at 37°C for 2 hours, followed by 5 washes in washing buffer. One hundred mL of biotinylated rat anti-mouse rIFN-g antibody diluted in sample buffer to 2 mg/mL, were added to each well and plates were incubated at 37°C for 1 hr followed by 5 washes with washing buffer. To each well, 100 mL of avidin-HRP (1/2000 dilution) were then added and plates were incubated for one hour at room temperature, followed by 5 washes with washing buffer.

Color reaction was developed by adding 100 mL of tetra methyl benzidine (TMB) /H₂O₂ and the reaction was terminated after 15 minutes by adding 50 mL of H₂SO₄. The plates were read at 450nm in a Multiscan plate reader and the results were calculated using the Titersoft data reduction program. For IL-5 detection, similar protocol was followed with appropriate antibodies and stanadards from Pharmingen and Biosource International.

Splenocytes were restimulated with BC or BCH4 cells at day 7 and then tested for CTL activity after 12 days. CTL assays were carried out as follows. Splenocytes (2.5 to 3 x 10⁶ cells/mL) were incubated in complete RPMI containing 10 U/mL IL-2 and 2.5-3 x 10⁶ cells/mL γ-irradiated (3,000 rads) syngeneic splenocytes which had been infected with 1 pfu/cell RSV for 2 hr at 37°C. After 5 days of culture, cells were washed and incubated (4 hr/37°C) at varying effector cell dilutions with 2 x 10^{3 51}Cr-labeled target cells (persistently RSV-infected BCH4 fibroblasts). Uninfected BALB/c fibroblasts (BC cells) served as a control. Spontaneous and total chromium releases were measured in supernatants of target cells suspended in medium or 2.5 % Triton-X 100. The percent of specific lysis was calculated as (counts - spontaneous counts)/(total counts - spontaneous counts) x 100. Results are reported as mean specific lysis from triplicate assays. Each experiment was performed 3 times.

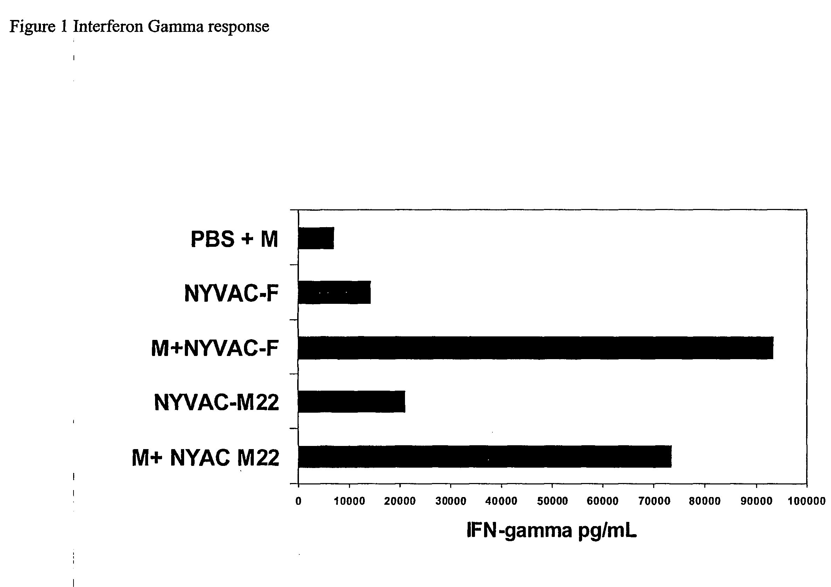

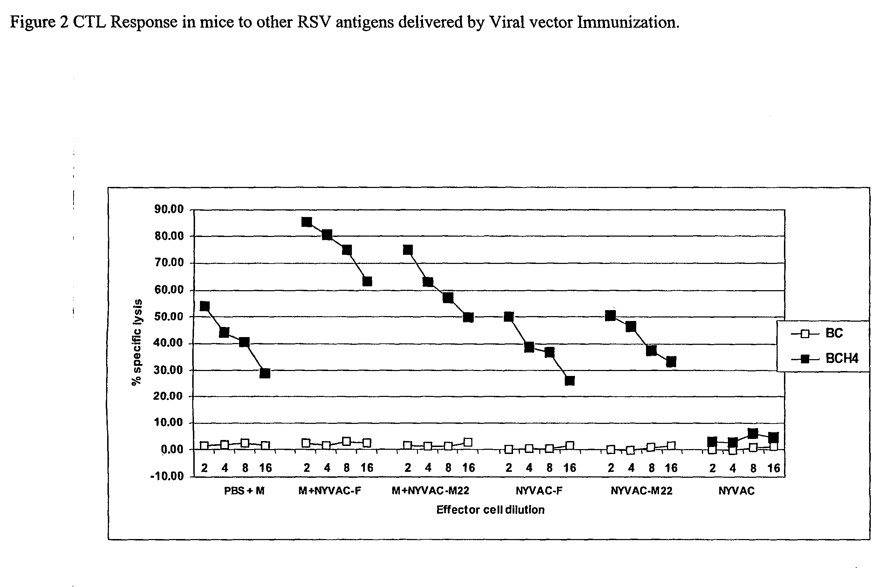

These experiments show that boosting with RSV M protein antigen enhances CD8 T-cell responses as measured by IFN-γ shown in Fig 1, and CTL activity shown in Fig 2. RSV M antigen can enhance the specific response to at least two RSV antigens tested, RSV F and M22.

### Example 3:

### This example illustrates the T-cell response in mice to other RSV antigens when RSV M protein is co-administered.

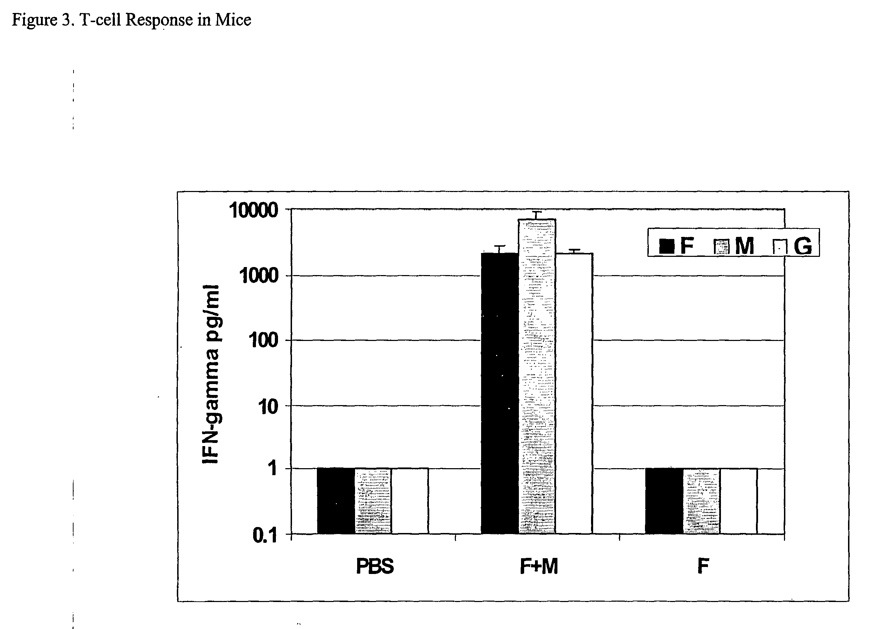

Groups of BALB/c mice were primed with 10³ pfu of live RSV intranassally as before. Four weeks after priming the mice were boosted with PBS, purified RSV F protein (50 ng) in aluminum phosphate or purified RSV F plus purified RSV M (1µg) in aluminum phosphate. Four weeks after boosting the spleens were harvested as before, cultured and incubated *in vitro* with purified RSV F, G or M proteins (0.5 µg/ml final). The cells were plated at 3x10⁶ cells/ml and were stimulated with the same number of syngeneic spleen cells γ-irradiated at 3000 rads. Supernatants were collected at 72 h and were assayed for IFN-γ as described in Example 2. The results of this assay are shown in Fig. 3. No detectable levels of anti-F, G or M responses were found in mice that were not boosted (PBS). Mice that were boosted with F plus M (F+M) responded to F, M and also G. The anti-G response is attributable to trace amounts of G present in the purified F. The mice that received only F boost (F) did not produce significant IFN-γ to any of the proteins. The IFN-γ response to RSV F in the F+M group of mice was enhanced when compared to the RSV F only boosted group.

### Example 4:

### This example illustrates the antibody response to other RSV proteins in mice when RSV M is co-administered.

Groups of BALB/c mice were primed with 10³ pfu of live RSV intranassally as before. Four weeks later the mice were boosted with either PBS, or FI-RSV (formalin-inactivated) 5 ng of F with 1 µg of M in alum (aluminum phosphate) or 5 ng of F in alum. The animals were boosted again four weeks later and the sera samples were collected to determine anti-F responses by F-specific ELISA.

The spleens were also collected and were restimulated with 500ng/ml of F, M, G or 10⁶PFU of RSV. The culture supernates were collected 72hrs later for IFN-gamma and IL-5 determinations.

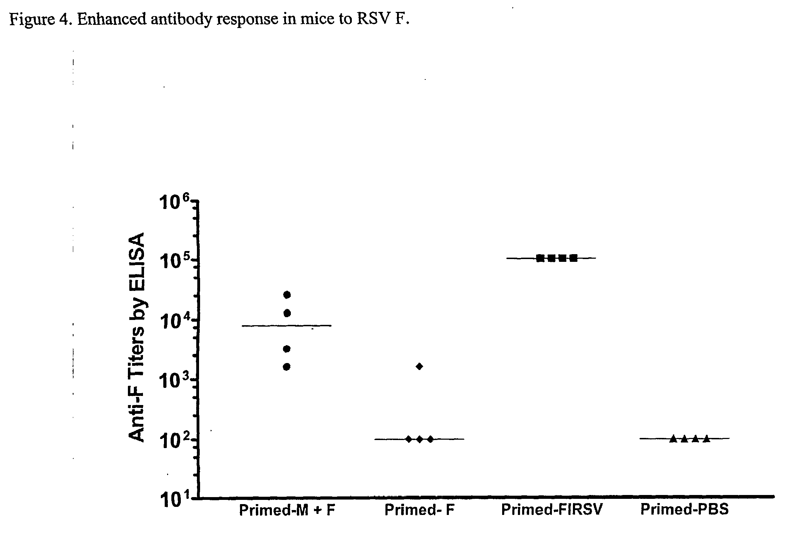

The antibody results shown in Fig. 4, show that the inclusion of the RSV M antigen enhances the antibody response (antibody titre) to RSV F antigen by two logs when compared to the RSV F only boost.

**Table 1.**

| Groups | Primary | Boost 1 | Total # of mice |
|---|---|---|---|
| 1 | RSV 10³pfu | PBS | 5 |
| 2 | RSV 10³pfu | M (1ug)+NYVAC-F | 5 |
| 3 | RSV 10³pfu | M (1ug)+NYVAC-M22 | 5 |
| 4 | RSV 10³pfu | NYVAC-F | 5 |
| 5 | RSV 10³pfu | NYVAC-M22 | 5 |
| 6 | RSV 10³pfu | M (1 ug)+NYVAC | 5 |
| 7 | PBS | PBS | 5 |
| 8 | PBS | M (lug)+NYVAC-F | 5 |
| 9 | PBS | M (1ug)+NYVAC-M22 | 5 |
| 10 | PBS | NYVAC-F | 5 |
| 11 | PBS | NYVAC-M22 | 5 |
| 12 | PBS | M(1ug)+NYVAC | 5 |

### REFERENCES

1. Robbins, A., and Freeman, P. (1988). Sci. Am. 259:126-133.
2. Hall CB. Prospects for a respiratory syncytial virus vaccine. (1994) Science 265:1393-1394.
3. Falsey AR, Cunningham CK, Barker WH, et al. Respiratory syncytial virus and influenza A infections in the hospitalized elderly. (1995) J. Infect. Dis. 172:389-394.
4. Nicholson KG, Kent J, Hammersley V, Cancio E. Acute viral infections of upper respiratory tract in elderly people living in the community: comparative, prospective, population based study of disease burden. (1997) BMJ 315:1060-1064.
5. Nicholson KG. Impact of influenza and respiratory syncytial virus on mortality in England and Wales from January 1975 to December 1990. Epidemiol Infect 1996;116:51-63.
6. Han L. L., Alexander J. P_, Anderson L. J. (1999) Vaccine *179:25-30.*
7. Collins, P., McIntosh, K., and Chanock, R.M., in "Fields of Virology" ed_ Fields, B.M. , Knipe, D.M_ and Howley P.M., Lippincott-Raven Press New York, *(1996) pp.* 1313-1351.
8. Coates, H.N., et al. (1966). Am. J. Epidemeol. 83259-313.
9. Johnson, RR., et al. (1987). J. Virol. 61:3163-3166.
10. Walsh, E.E., Hall, C.B., Briselli, M., Braudiss, M.W., and Schlesinger, J.J. *(1987). J. Infec. Dis.* 155:1198-1204.
11. Walsh, E.E., Hruska, J. (1983). J. Virol. 47_171-177.
12. Levine, S., Kleiber-France, R, and Paradiso, *P.R. (1987) J.* Gen- Virol. 69:2521-2524.
13. Anderson, L.J., *Hierholzer, J.C.,* Tsou, C., Hendry, R.M., Fernie, B.F., Stone Y_ and McIntosh, K. (1985) J. Infec. Dis.151:626-633.
14. Wertz, G.W., Sullender, W.M. (1992) Biotech *20*:151-176.
15. Cherrie, A.H., Anderson, K., Wertz, G.W. and Openshaw, P.J.M. (1992) J. Virol. 66:2102-2110.
16. Collins, P.L., Huang, Y.T. and Wertz, G.W. (1984). Proc. Nat). Acad. Sci. 81:7683-7687.
17. Wertz, G.W., Collins P.L., Huang, Y., Gruber C., Levine S., and Ball, L.A. (1985) Proc. Natl. Acad. Sci. 82:4075-4079.
18. Satake, M. and Venkatesan S. (1984). J.Virol. 50:92-99.
19. Levine S, Dillman TR, and Montgomery PC (1989). Proc. Soc. Exp. Biol Med 190:349-356.
20. Paoletti E, Tartaglia J, Taylor J. (1994) Dev Biol Stand 82:65-69.

## Claims

1. An immunogenic composition comprising purified M protein from respiratory syncytial virus or at least one immunoeffective fragment thereof and a nucleic acid vector encoding a respiratory syncytial virus antigen or at least one immunogenic fragment thereof, wherein said M protein or immunoeffective fragment is provided in an amount to provide an enhanced immune response to said respiratory syncytial virus antigen in a host having a pre-existing respiratory syncytial virus M-specific immune response, wherein the nucleic acid vector comprises an antigen-encoding portion and a promoter to effect expression of the antigen-encoding portion in the host.

2. A composition of Claim 1 which is formulated as a vaccine, optionally containing a pharmaceutically-acceptable carrier.

3. A composition of either Claim 1 or Claim 2 wherein said respiratory syncytial virus antigen is selected from a respiratory syncytial virus G antigen free from respiratory syncytial virus F antigen, respiratory syncytial virus F antigen free from respiratory syncytial virus G antigen, or a respiratory syncytial virus M22 antigen.

4. A composition of any preceding claim wherein said pre-existing respiratory syncytial virus M-specific immune response is a T-cell immune response.

5. A composition of any preceding claim wherein the amount of purified M protein is 0.1 µg to 1,000 µg per dose.

6. A composition of any preceding claim wherein the amount of purified M protein is 1 µg to 100 µg per dose.

7. A composition of any preceding claim wherein the amount of purified M protein is 1 µg to 50 µg per dose.

8. A composition of any preceding claim wherein the purified M protein is greater than 50% pure as measured by SDS-PAGE analysis or in an M-specific ELISA assay.

9. A method of making an immunogenic composition comprising providing purified M protein from respiratory syncytial virus or at least one immunoeffective fragment thereof and a nucleic acid encoding a respiratory syncytial virus antigen, wherein said M protein or immunoeffective fragment is provided in an amount to provide an enhanced immune response to said respiratory syncytial virus antigen in a host having a pre-existing respiratory syncytial virus M-specific immune response.

10. A method of Claim 9, wherein said respiratory syncytial virus antigen is selected from a respiratory syncytial virus G antigen free from respiratory syncytial virus F antigen, respiratory syncytial virus F antigen free from respiratory syncytial virus G antigen, or a respiratory syncytial virus M22 antigen.

11. Use of a purified M protein or immunogenic component thereof from respiratory syncytial virus as an adjuvant for the preparation of an immunogenic composition for use in a host having a pre-existing respiratory syncytial virus M-specific immune response, wherein said composition comprises a respiratory syncytial virus antigen and said M-protein.

12. Use of Claim 11, wherein said antigen is selected from a respiratory syncytial virus G antigen free from respiratory syncytial virus F antigen, respiratory syncytial virus F antigen free from respiratory syncytial virus G antigen, or a respiratory syncytial virus M22 antigen.

13. Use of a purified M protein from respiratory syncytial virus, or immunoeffective fragments thereof, for the manufacture of a medicament comprising a nucleic and vector encoding a respiratory syncytial virus antigen for enhancing the immune response to a respiratory syncytial virus antigen in a host having a pre-existing respiratory syncytial virus M-specific immune response.

14. Use of Claim 13, wherein said antigen is selected from a respiratory syncytial virus G antigen free from respiratory syncytial virus F antigen, respiratory syncytial virus F antigen free from respiratory syncytial virus G antigen, or a respiratory syncytial virus M22 antigen.

15. Use of Claim 13 for treating in said host a disease caused by respiratory syncytical virus, wherein said antigen is a respiratory syncytial virus G antigen, a respiratory syncytial virus F antigen or a respiratory syncytial virus M22 antigen.

16. Use of Claim 15 wherein said manufacture comprises:
i) purifying M protein of respiratory syncytial virus;
ii) mixing a pre-selected amount of said purified M protein with a nucleic acid vector encoding a respiratory syncytial virus antigen; and
iii) formulating said mixture as a vaccine.

## Patentansprüche

1. Eine immunogene Zusammensetzung, welche gereinigtes M-Protein aus dem respiratorischen Synzytialvirus oder wenigstens ein immunologisch wirksames Fragment von diesem und einen Nukleinsäurevektor, welcher ein Antigen des respiratorischen Synzytialvirus oder wenigstens ein immunogenes Fragment von diesem codiert, umfasst, wobei das M-Protein oder das immunologisch wirksame Fragment in einer Menge bereitgestellt wird, um eine verstärkte Immunantwort auf das Antigen des respiratorischen Synzytialvirus in einem Wirt mit einer vorher bestehenden respiratorisches Synzytialvirus M-spezifischen Immunantwort zu liefern, wobei der Nukleinsäurevektor einen antigencodierenden Bereich und einen Promoter umfasst, um die Expression des antigencodierenden Bereichs in dem Wirt zu bewirken.

2. Eine Zusammensetzung nach Anspruch 1, welche als ein Impfstoff formuliert ist, die gegebenenfalls einen pharmazeutisch verträglichen Träger enthält.

3. Eine Zusammensetzung nach entweder Anspruch 1 oder Anspruch 2, wobei das Antigen des respiratorischen Synzytialvirus ausgewählt ist aus einem G-Antigen des respiratorischen Synzytialvirus, das frei ist von F-Antigen des respiratorischen Synzytialvirus, einem F-Antigen des respiratorischen Synzytialvirus, das frei ist von G-Antigen des respiratorischen Synzytialvirus, oder einem M22-Antigen des respiratorischen Synzytialvirus.

4. Eine Zusammensetzung nach irgendeinem vorhergehenden Anspruch, wobei die vorher bestehende respiratorisches Synzytialvirus M-spezifische Immunantwort eine T-Zell-Immunantwort ist.

5. Eine Zusammensetzung nach irgendeinem vorhergehenden Anspruch, wobei die Menge an gereinigtem M-Protein 0,1 µg bis 1.000 µg pro Dosis beträgt.

6. Eine Zusammensetzung nach irgendeinem vorhergehenden Anspruch, wobei die Menge an gereinigtem M-Protein 1 µg bis 100 µg pro Dosis beträgt.

7. Eine Zusammensetzung nach irgendeinem vorhergehenden Anspruch, wobei die Menge an gereinigtem M-Protein 1 µg bis 50 µg pro Dosis beträgt.

8. Eine Zusammensetzung nach irgendeinem vorhergehenden Anspruch, wobei das gereinigte M-Protein zu mehr als 50% rein ist, wie durch SDS-PAGE-Analyse oder in einem M-spezifischen ELISA-Assay gemessen wird.

9. Ein Verfahren zur Erzeugung einer immunogenen Zusammensetzung, umfassend das Bereitstellen von gereinigtem M-Protein aus dem respiratorischen Synzytialvirus oder wenigstens eines immunologisch wirksamen Fragmentes von diesem und einer Nukleinsäure, welche ein Antigen des respiratorischen Synzytialvirus codiert, wobei das M-Protein oder das immunologisch wirksame Fragment in einer Menge bereitgestellt wird, um eine verstärkte Immunantwort auf das Antigen des respiratorischen Synzytialvirus in einem Wirt mit einer vorher bestehenden respiratorisches Synzytialvirus M-spezifischen Immunantwort zu liefern.

10. Ein Verfahren nach Anspruch 9, wobei das Antigen des respiratorischen Synzytialvirus ausgewählt wird aus einem G-Antigen des respiratorischen Synzytialvirus, das frei ist von F-Antigen des respiratorischen Synzytialvirus, einem F-Antigen des respiratorischen Synzytialvirus, das frei ist von G-Antigen des respiratorischen Synzytialvirus, oder einem M22-Antigen des respiratorischen Synzytialvirus.

11. Verwendung eines gereinigten M-Proteins oder einer immunogenen Komponente von diesem aus dem respiratorischen Synzytialvirus als ein Hilfsmittel zur Herstellung einer immunogenen Zusammensetzung zur Verwendung in einem Wirt, welcher eine vorher bestehende respiratorisches Synzytialvirus M-spezifische Immunantwort aufweist, wobei die Zusammensetzung ein Antigen des respiratorischen Synzytialvirus und das M-Protein umfasst.

12. Verwendung nach Anspruch 11, wobei das Antigen ausgewählt wird aus einem G-Antigen des respiratorischen Synzytialvirus, das frei ist von F-Antigen des respiratorischen Synzytialvirus, einem F-Antigen des respiratorischen Synzytialvirus, das frei ist von G-Antigen des respiratorischen Synzytialvirus, oder einem M22-Antigen des respiratorischen Synzytialvirus.

13. Verwendung eines gereinigten M-Proteins aus dem respiratorischen Synzytialvirus oder von immunologisch wirksamen Fragmenten von diesem zur Herstellung eines Medikaments, welches einen Nukleinsäurevektor umfasst, welcher ein Antigen des respiratorischen Synzytialvirus codiert, zur Verstärkung der Immunantwort auf ein Antigen des respiratorischen Synzytialvirus in einem Wirt mit einer vorher bestehenden respiratorisches Synzytialvirus M-spezifischen Immunantwort.

14. Verwendung nach Anspruch 13, wobei das Antigen ausgewählt wird aus einem G-Antigen des respiratorischen Synzytialvirus, das frei ist von F-Antigen des respiratorischen Synzytialvirus, einem F-Antigen des respiratorischen Synzytialvirus, das frei ist von G-Antigen des respiratorischen Synzytialvirus, oder einem M22-Antigen des respiratorischen Synzytialvirus.

15. Verwendung nach Anspruch 13 zur Behandlung einer Erkrankung, welche durch das respiratorische Synzytialvirus verursacht wird, in dem Wirt, wobei das Antigen ein G-Antigen des respiratorischen Synzytialvirus, ein F-Antigen des respiratorischen Synzytialvirus oder ein M22-Antigen des respiratorischen Synzytialvirus ist.

16. Verwendung nach Anspruch 15, wobei die Herstellung umfasst:
i) Reinigen von M-Protein des respiratorischen Synzytialvirus;
ii) Mischen einer vorher ausgewählten Menge des gereinigten M-Proteins mit einem Nukleinsäurevektor, welcher ein Antigen des respiratorischen Synzytialvirus codiert; und
iii) Formulieren der Mischung als einen Impfstoff.

## Revendications

1. Composition immunogène comprenant une protéine M purifiée provenant du virus respiratoire syncytial, ou au moins un fragment immunogène de celle-ci, et un vecteur d'acide nucléique codant un antigène du virus respiratoire syncytial, ou au moins un fragment immunogène de celui-ci, ladite protéine M ou ledit fragment immunogène étant fourni en une quantité susceptible d'induire une réponse immunitaire accrue vis-à-vis dudit antigène du virus respiratoire syncytial chez un hôte présentant une réponse immunitaire préexistante spécifique de la protéine M du virus respiratoire syncytial, et le vecteur d'acide nucléique comprenant un fragment codant d'un antigène et un promoteur permettant l'expression du fragment codant d'antigène chez l'hôte.

2. Composition selon la revendication 1, formulée sous la forme d'un vaccin, contenant éventuellement un support pharmaceutiquement acceptable.

3. Composition selon la revendication 1 ou la revendication 2, dans laquelle ledit antigène du virus respiratoire syncytial est choisi parmi un antigène G du virus respiratoire syncytial exempt de l'antigène F du virus respiratoire syncytial, un antigène F du virus respiratoire syncytial exempt de l'antigène G du virus respiratoire syncytial, ou un antigène M22 du virus respiratoire syncytial.

4. Composition selon l'une quelconque des revendications précédentes, dans laquelle ladite réponse immunitaire spécifique de la protéine M du virus respiratoire syncytial préexistante est une réponse immunitaire des lymphocytes T.

5. Composition selon l'une quelconque des revendications précédentes, la quantité de protéine M purifiée étant comprise entre 0,1 µg et 1 000 µg par dose.

6. Composition selon l'une quelconque des revendications précédentes, la quantité de protéine M purifiée étant comprise entre 1 µg et 100 µg par dose.

7. Composition selon l'une quelconque des revendications précédentes, la quantité de protéine M purifiée étant comprise entre 1 µg et 50 µg par dose.

8. Composition selon l'une quelconque des revendications précédentes, le degré de pureté de la protéine M purifiée, mesurée par une analyse en SDS-PAGE ou dans un test ELISA spécifique de la protéine M, étant supérieur à 50%.

9. Procédé de préparation d'une composition immunogène comprenant la fourniture d'une protéine M purifiée provenant du virus respiratoire syncytial, ou au moins un fragment immunogène de celle-ci, et un acide nucléique codant un antigène du virus respiratoire syncytial, ladite protéine M ou ledit fragment immunogène étant fourni en une quantité susceptible d'induire une réponse immunitaire accrue à l'égard dudit antigène du virus respiratoire syncytial chez un hôte présentant une réponse immunitaire préexistante spécifique de la protéine M du virus respiratoire syncytial.

10. Procédé selon la revendication 9, ledit antigène du virus respiratoire syncytial étant choisi parmi un antigène G du virus respiratoire syncytial exempt de l'antigène F du virus respiratoire syncytial, un antigène F du virus respiratoire syncytial exempt de l'antigène G du virus respiratoire syncytial, ou un antigène M22 du virus respiratoire syncytial.

11. Utilisation d'une protéine M purifiée provenant du virus respiratoire syncytial, ou d'un composant immunogène de celle-ci, en tant qu'adjuvant pour la préparation d'une composition immunogène destinée à être utilisée chez un hôte présentant une réponse immunitaire spécifique de la protéine M du virus respiratoire syncytial préexistante, ladite composition comprenant un antigène du virus respiratoire syncytial et ladite protéine M.

12. Utilisation selon la revendication 11, ledit antigène étant sélectionné parmi un antigène G du virus respiratoire syncytial exempt de l'antigène F du virus respiratoire syncytial, un antigène F du virus respiratoire syncytial exempt de l'antigène G du virus respiratoire syncytial, ou un antigène M22 du virus respiratoire syncytial.

13. Utilisation d'une protéine M purifiée provenant du virus respiratoire syncytial, ou de composants immunogènes de celle-ci, pour la préparation d'un médicament comprenant un vecteur d'acide nucléique codant un antigène du virus respiratoire syncytial destiné à amplifier la réponse immunitaire à l'encontre d'un antigène du virus respiratoire syncytial chez un hôte présentant une réponse immunitaire spécifique de la protéine M du virus respiratoire syncytial préexistante.

14. Utilisation selon la revendication 13, ledit antigène étant sélectionné parmi un antigène G du virus respiratoire syncytial exempt de l'antigène F du virus respiratoire syncytial, un antigène F du virus respiratoire syncytial exempt de l'antigène G du virus respiratoire syncytial, ou un antigène M22 du virus respiratoire syncytial.

15. Utilisation selon la revendication 13 destinée au traitement chez ledit hôte d'une maladie provoquée par le virus respiratoire syncytial, ledit antigène étant un antigène G du virus respiratoire syncytial, un antigène F du virus respiratoire syncytial ou un antigène M22 du virus respiratoire syncytial.

16. Utilisation selon la revendication 15, ladite fabrication comprenant :
i) la purification d'une protéine M du virus respiratoire syncytial ;
ii) le mélange d'une quantité prédéterminée de ladite protéine M purifiée avec un vecteur d'acide nucléique codant un antigène du virus respiratoire syncytial, et
iii) la formulation dudit mélange sous la forme d'un vaccin.
